(19) Europäisches Patentamt
European Patent Office
Office européen des brevets

(11) Numéro de publication : **0 376 801 B1**

# (12) FASCICULE DE BREVET EUROPEEN

(45) Date de publication du fascicule du brevet : **01.07.92 Bulletin 92/27**

(21) Numéro de dépôt : **89403549.2**

(22) Date de dépôt : **19.12.89**

(51) Int. Cl.$^5$ : **C07C 229/12,** C07C 237/06, C07C 327/06, C07C 227/08, C07C 233/13, C07C 69/63, C11D 1/60, C11D 1/62

## (54) Acétates, thioacétates et acétamides de polyfluoroalkyle substitués, leur procédé de préparation et leurs applications.

(30) Priorité : **27.12.88 FR 8817240**

(43) Date de publication de la demande : **04.07.90 Bulletin 90/27**

(45) Mention de la délivrance du brevet : **01.07.92 Bulletin 92/27**

(84) Etats contractants désignés : **BE CH DE ES FR GB IT LI**

(56) Documents cités :
**EP-A- 0 152 784**
**DE-A- 1 953 279**
**US-A- 3 510 494**
**US-A- 4 049 668**
**US-A- 4 107 055**

(73) Titulaire : **ELF ATOCHEM S.A.**
**4 & 8, Cours Michelet La Défense 10**
**F-92800 Puteaux (FR)**

(72) Inventeur : **Sismondi, Alain**
**25 Avenue Jean de la Fontaine La Ségurana**
**Bâtiment 2 F-06100 Nice (FR)**
Inventeur : **Abenin, Parfait**
**25 Avenue Jean de la Fontaine La Ségurana**
**Bâtiment 2 F-06100 Nice (FR)**
Inventeur : **Cambon, Aimé**
**59 Parc Mosca**
**F-06000 Nice (FR)**

(74) Mandataire : **Leboulenger, Jean et al**
**ATOCHEM Département Propriété Industrielle**
**Cédex 42- La Défense 10**
**F-92091 Paris la Défense (FR)**

**EP 0 376 801 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne de nouveaux composés polyfluorés et a plus particulièrement pour objet des composés substitués du type acétate, thioacétate ou acétamide, utilisables notamment comme agents tensio-actifs ou comme "monomères" pour l'élaboration de vésicules artificielles.

On connaît déjà de nombreux tensio actifs fluorés et en particulier des sels d'ammonium quaternaire dans lesquels un radical perfluoré est relié au groupement ammonium quaternaire (par exemple trialkylammonium ou pyridinium) par un pont dont la nature influe grandement sur les propriétés applicatives. Ce pont peut être très simple, par exemple $CH_2$ ou $C_2H_4$ (brevets US 2 727 923 et FR 1 588 482) ou plus complexe, par exemple $-C_2H_4SO_2NH(CH_2)_3-$ (brevet FR 2 084 888), $-C_2H_4S(CH_2)_3-OCH_2CH(OH)CH_2-$ (brevet EP 256 980), etc...

La présente invention a maintenant pour objet une nouvelle famille de composés polyfluorés qui peuvent être représentés par la formule générale :

$$Rf - (CH_2)_m - Q - \overset{O}{\overset{\|}{C}} - CH_2 - \overset{+}{N}(R_1)(R_2)(R_3) \quad X^- \qquad (I)$$

dans laquelle Rf représente un radical perfluoroalkyle, linéaire ou ramifié, contenant de 2 à 16 atomes de carbone, m est un nombre entier allant de 1 à 4, de préférence égal à 2, Q désigne un atome d'oxygène, de soufre ou un groupe NH, $X^-$ représente un anion monovalent ou son équivalent, $R_1$ représente un radical alkyle contenant de 1 à 3 atomes de carbone, $R_2$ représente le radical allyle ou un radical alkyle, linéaire ou ramifié, contenant de 1 à 18 atomes de carbone, et $R_3$ représente un radical alkyle, linéaire ou ramifié, contenant de 7 à 18 atomes de carbone ou l'un des radicaux suivants :

$$-(CH_2)_n-S-(CH_2)_m-Rf' \qquad (II)$$

$$-(CH_2)_n-O-CO-C(R) = CH_2 \qquad (III)$$

$$- Y-\overset{+}{N}(R_1)(R_2) — CH_2-CO-Q-(CH_2)_m-Rf \quad X^- \qquad (IV)$$

dans lesquels les symboles m, $R_1$, $R_2$, Q, Rf et $X^-$ sont tels que définis ci-dessus, n est un nombre entier allant de 2 à 4, R représente un atome d'hydrogène ou un radical méthyle, Y désigne un pont alkylène de 2 à 8 atomes de carbone éventuellement interrompu par un atome d'oxygène, et Rf' représente un radical perfluoroalkyle, linéaire ou ramifié, contenant de 2 à 16 atomes de carbone. Les radicaux Rf et Rf' peuvent être identiques ou différents et sont de préférence des radicaux perfluoroalkyle linéaires contenant 4, 6, 8 ou 10 atomes de carbone.

Les composés de formule (I) peuvent être préparés à partir des halogèno-acétates, -thioacétates et -acétamides de formule :

$$Rf-(CH_2)_m-Q-\overset{O}{\overset{\|}{C}}-CH_2-Cl \text{ (ou Br)} \qquad (V)$$

dont certains sont connus (brevets FR 1 438 617 et US 4 107 055) et qu'on peut obtenir par réaction d'un halogénure d'halogénoacétyle de formule :

$$ZCH_2COZ' \qquad (VI)$$

où Z et Z', identiques ou différents, désignent chacun atome de chlore ou de brome sur un polyfluoroalcanol $Rf-(CH_2)_m-OH$, un polyfluoroalcanethiol $Rf(CH_2)_m-SH$ ou une polyfluoroalkylamine $Rf-(CH_2)_m-NH_2$. Les deux réactifs peuvent être utilisés en quantités équimolaires, mais il est généralement avantageux d'employer un léger excès d'halogénure (VI).

Lorsqu'on part d'un polyfluoroalcanol ou d'un polyfluoroalcanethiol pour préparer les composés de formule (V) dans lesquels Q représente respectivement un atome d'oxygène ou de soufre, la réaction peut être effectuée à une température comprise entre 20 et 70°C, sous atmosphère de gaz inerte, en l'absence de solvant

ou au sein d'un solvant organique anhydre, par exemple un éther, un hydrocarbure halogéné ou l'acétonitrile. Cependant, l'addition des réactifs doit se faire en refroidissant le milieu à une température comprise entre -5 et +10°C, de préférence entre environ 0 et 5°C. Le réacteur doit en outre être muni d'un dispositif permettant de piéger l'acide chlorhydrique ou bromhydrique dégagé durant la réaction. A nombre d'atomes de carbone équivalent, la réaction est plus rapide avec un polyfluoroalcane-thiol qu'avec un polyfluoroalcanol. Les halogéno-2 acétates ou thioacétates de polyfluoroalkyle ainsi obtenus peuvent être utilisés tels quels ou purifiés par distillation, si nécessaire.

Lorsqu'on part d'une polyfluoroalkylamine pour préparer les composés (V) dans lesquels Q représente un groupe NH, la réaction est effectuée en présence d'une base permettant de fixer l'acide chlorhydrique ou bromhydrique formé. Comme bases, on peut utiliser par exemple les amines tertiaires telles que la triéthylamine et la pyridine, les carbonates alcalins et les hydroxydes alcalins. La réaction peut être effectuée à une température comprise entre 20 et 50°C, sous atmosphère de gaz inerte, au sein d'un solvant organique anhydre, par exemple un éther, un hydrocarbure halogéné ou un hydrocarbure aromatique. Comme dans le cas des polyfluoroalcanols et des polyfluoroalcane-thiols, l'addition des réactifs doit se faire en refroidissant le milieu réactionnel à une température comprise entre -5 et +10°C, de préférence entre 0 et5°C environ. Les N-polyfluoroalkyl halogéno-2 acétamides ainsi obtenus peuvent être utilisés tels quels ou après recristallisation, par exemple dans l'éther de pétrole.

Les polyfluoroalcanols $Rf-(CH_2)_m-OH$, les polyfluoroalcane-thiols $Rf-(CH_2)_m-SH$ et les polyfluoroalkylamines $Rf-(CH_2)_m-NH_2$ sont des composés bien connus. Voir par exemple les brevets suivants :

– Polyfluoroalcanols : FR 1 380 579, US 3 083 224, US 3 145 222 et US 3 285 975
– Polyfluoroalcane-thiols : US 2 894 991, US 3 088 849, US 3 544 663, FR 1 221 415 et FR 2 083 422
– Polyfluoroalkylamines : DE 1 768 939, FR 1 532 284, JP 77 118406 et US 3 257 407.

Les composés de formule (V) sont extrêmement réactifs vis-à-vis des nucléophiles. Ils permettent les réactions de substitution, sans risque de subir une réaction d'élimination conduisant à la formation de sous-produits insaturés.

Les composés de formule (I) selon l'invention peuvent être préparés par réaction d'un composé de formule (V) sur l'amine tertiaire correspondante. La synthèse est réalisée en utilisant des quantités stoechiométriques de composé (V) et de l'amine choisie, ces réactifs pouvant éventuellement être dissous dans 2 à 3 volumes d'éther. Il se produit une réaction exothermique avec formation d'un bromure ou chlorure d'ammonium quaternaire. Le temps de réaction et l'aspect physique des sels formés varient selon la nature de l'amine choisie. Tous ces sels sont solubles dans l'eau et la plupart ont une structure cristalline.

Comme exemples non limitatifs d'amines utilisables, on peut citer les N,N-diméthyl-alkylamines dont le radical alkyle contient de 7 à 18 atomes de carbone, les N-méthyl-dialkylamines dont les radicaux alkyle contiennent de 7 à 18 atomes de carbone, les N-méthyl-N-allyl-alkylamines dont le radical alkyle contient de 7 à 18 atomes de carbone, les N,N-diméthyl-aminoalkyl acrylates et méthacrylates, ainsi que les amines fluorées du type $Rf'-(CH_2)_m-S-(CH_2)_n-N(CH_3)_2$ qui conduisent aux sels d'ammonium quaternaire de formule :

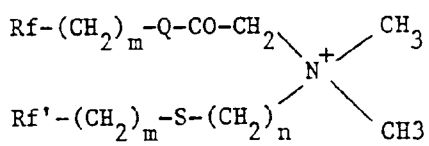

$X^-$

dans laquelle Q, m, n, Rf, Rf' et X ont les mêmes significations que précédemment.

Un exemple de diamine tertiaire utilisable est la N,N,N',N'-tétraméthyl éthylènediamine qui conduit à des sels doubles de formule :

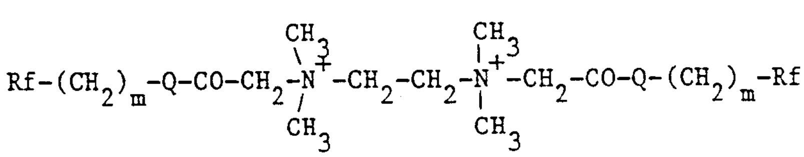

, $2X^-$

D'autres exemples de diamines tertiaires utilisables sont la N,N,N',N'-tétraméthyl hexanediamine-1,6 et le N,N,N',N'-tétraméthyl diaminodiéthyléther.

L'anion bromure ou chlorure des sels d'ammonium peut facilement être échangé contre un autre anion par des méthodes bien connues en soi. Comme exemples d'autres anions on peut mentionner les anions iodure, nitrate, paratoluènesulfonate, sulfate, alkylsulfate, picrate.

Les sels d'ammonium selon l'invention sont de précieux agents tensio-actifs, utilisables comme additifs dans des domaines très variés en tant qu'agents mouillant, émulsifiants, dispersants ou moussants ; ils sont par ailleurs utilisables comme monomères pour l'élaboration de vésicules artificielles.

Les exemples suivants illustrent l'invention sans la limiter. Les exemples 1 à 4 concernent la préparation des intermédiaires de formule (V) et les exemples 5 et 6 celle des sels d'ammonium selon l'invention.

## EXEMPLE 1

Dans un réacteur équipé d'un réfrigérant, d'une ampoule à brome et d'un dispositif permettant de piéger HBr, on place 0,01 mole (2,64 g) de perfluorobutyl-2 éthanol $C_4F_9$-$C_2H_4$-OH. Après avoir purgé le montage à l'azote et refroidi le réacteur à 0°C, on additionne lentement par l'ampoule à brome 0,011 mole (2,22 g) de bromure d'acide bromoacétique Br-$CH_2$-COBr préalablement dissout dans 15 ml d'éther éthylique anhydre. On chauffe ensuite pendant 24 heures à 60°C sous atmosphère inerte, puis le mélange est hydrolysé et extrait à l'éther éthylique. Les phases éthérées sont abondamment rincées jusqu'à ce que l'eau de lavage ait un pH neutre. La phase organique est ensuite séchée sur sulfate de sodium anhydre, puis le solvant est évaporé et le résidu est distillé. On obtient ainsi avec un rendement de 69 % le bromoacétate de perfluorobutyl-2 éthyle $C_4F_9$-$C_2H_4$-O-CO-$CH_2$-Br qui bout à 60°C sous 67 Pa.

En opérant de la même façon à partir de l'alcool $C_6F_{13}$-$C_2H_4$-OH on obtient le bromoacétate de perfluorohexyl-2 éthyle $C_6F_{13}$-$C_2H_4$-O-CO-$CH_2$-Br qui bout à 74°C sous 67 Pa. Rendement : 68 %.

## EXEMPLE 2

On opère comme à l'exemple 1, mais en remplaçant le perfluorobutyl-2 éthanol par 0,01 mole de perfluorobutyl-2 éthanethiol $C_4F_9C_2H_4SH$ et en chauffant seulement pendant 12 heures à 40°C au lieu de 24 heures à 60°C.

On obtient ainsi avec un rendement de 70 % le bromothioacétate de perfluorobutyl-2 éthyle $C_4F_9C_2H_4$-S-CO-$CH_2$-Br qui bout à 61°C sous 133 Pa.

En opérant de la même façon à partir des thiols $C_6F_{13}C_2H_4SH$ et $C_8F_{17}C_2H_4SH$, on obtient respectivement les bromothioacétates suivants :

| | | |
|---|---|---|
| $C_6F_{13}C_2H_4$-S-CO-$CH_2$-Br | Eb. 90°C/133 Pa | Rdt : 68 % |
| $C_8F_{17}C_2H_4$-S-CO-$CH_2$-Br | Eb. 118°C/133 Pa | Rdt : 72 % |

## EXEMPLE 3

Dans un réacteur équipé d'un réfrigérant et d'une ampoule à brome, on place 0,015 mole de chlorure d'acide chloroacétique Cl-$CH_2$-COCl en solution dans 10 ml d'éther éthylique anhydre. Le réacteur étant refroidi à 0°C, on additionne par l'ampoule à brome 0,01 mole de perfluorohexyl-2 éthylamine $C_6F_{13}$-$C_2H_4$-$NH_2$ et 0,01 mole de pyridine en solution dans 20 ml d'éther éthylique anhydre. Après retour à température ambiante, on chauffe pendant 24 heures à 40°C sous atmosphère inerte. Le mélange réactionnel est ensuite acidifié avec une solution d'acide chlorhydrique à 50 %, puis extrait à l'éther éthylique. Les phases organiques sont séchées sur sulfate de sodium anhydre, puis l'éther est évaporé et le résidu est distillé sous pression réduite ou recristallisé dans l'éther de pétrole. On obtient ainsi le chloroacétamide de perfluorohexyl-2 éthyle : $C_6F_{13}$-$C_2H_4$-NH-CO-$CH_2$-Cl qui fond à 30°C. Rendement : 65 %.

## EXEMPLE 4

On opère comme à l'exemple 3 mais en remplaçant le chlorure d'acide chloroacétique Cl-$CH_2$-COCl par son homologue bromé Br-$CH_2$-COBr. On obtient le bromoacétamide suivant :

$C_6F_{13}$-$C_2H_4$-NH-CO-$CH_2$-Br  Eb = 85°C/133 Pa  Rdt = 70 %

Si on remplace en outre la perfluorohexyl-2 éthylamine par la perfluorobutyl-2 éthylamine, on obtient le bromoacétamide suivant :

$C_4F_9$-$C_2H_4$-NH-CO-$CH_2$-Br  Eb = 80°C/133 Pa  Rdt = 76 %

EXEMPLE 5

A partir des composés bromés décrits aux exemples 1 (bromoacétates), 2 (bromothioacétates) et 4 (bromoacétamides) et des amines $NR_1R_2R_3$ mentionnées dans la seconde colonne des tableaux (A), (B) et (C), on a préparé les sels de formule :

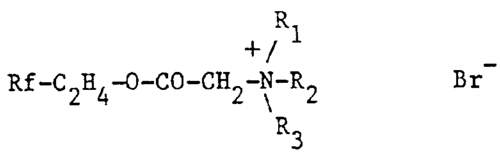

(I-a)

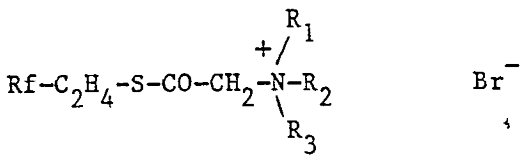

(I-b)

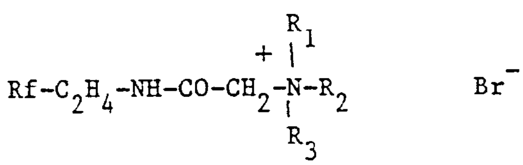

(I-c)

en procédant comme suit :

Dans un réacteur équipé d'un réfrigérant et muni d'un agitateur magnétique, on introduit des quantités équimolaires d'amine $NR_1R_2R_3$ et de composé bromé dissous dans 2 à 3 volumes d'éther éthylique. Selon la nature des réactifs plusieurs cas se présentent :

Le plus souvent, on observe une précipitation immédiate sous forme d'un solide blanc. La réaction est poussée à son terme en chauffant au reflux de l'éther jusqu'à consommation totale du bromure. Le précipité est ensuite filtré, rinçé à l'éther éthylique et séché.

Pour certaines combinaisons de réactifs, un trouble apparaît très rapidement. On laisse la solution reposer et on récupère par décantation une huile plus ou moins visqueuse qui est rinçée à l'éther éthylique anhydre.

Pour quelques autres enfin, on n'observe ni précipité ni trouble apparent. Cependant, si on abaisse la température entre 0 et 10°C, il apparaît un gel qui se gonfle de solvant. Après évaporation du solvant, on obtient l'ammonium attendu sous la forme d'un solide ou d'une huile.

Le tableau A correspond aux sels d'ammonium de formule (I-a) préparés à partir des bromoacétates de l'exemple 1, le tableau B aux sels de formule (I-b) préparés à partir des bromothioacétates de l'exemple 2, et le tableau C aux sels de formule (I-c) préparés à partir des bromoacétamides de l'exemple 4, la première colonne de ces tableaux précisant la nature du radical perfluoroalkyle du composé bromé utilisé.

Les quatrième et cinquième colonnes de ces tableaux indiquent respectivement en mN/m les tensions superficielle $\gamma s$ et interfaciale $\gamma i$ (cyclohexane) du sel d'ammonium considéré, mesurées à 25°C en solution aqueuse à 0,1 %, sauf indication contraire signalée entre parenthèses par l'une des lettres suivantes :

i : solution aqueuse à 0,2 %
j : " " à 0,3 %
k : " " à 0,5 %
l : " " à 1 %

TABLEAU A

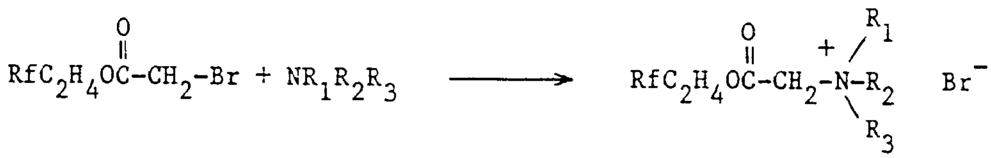

(I-a)

| Rf | Amine $NR_1R_2R_3$ | COMPOSE I-a<br>Rendement (%) | γs | γi |
|---|---|---|---|---|
| $C_4F_9$ | N,N-diméthyl heptylamine | 95 | 16,7 (l) | 1,9 (l) |
| $C_6F_{13}$ | idem | 97 | 15,3 (j) | |
| $C_4F_9$ | N,N-diméthyl octylamine | 95 | 16,6 (j) | |
| $C_6F_{13}$ | idem | 93 | 15,8 (j) | |
| $C_4F_9$ | N,N-diméthyl nonylamine | 95 | 16,7 (j) | |
| $C_6F_{13}$ | idem | 93 | 15,5 (j) | 2,4 (j) |

TABLEAU A (suite)

| Rf | Amine $NR_1R_2R_3$ | Rendement (%) | COMPOSE I-a γs | γi |
|---|---|---|---|---|
| $C_4F_9$ | N,N-diméthyl décylamine | 97 | 17,5 (j) | |
| $C_6F_{13}$ | idem | 94 | 15,7 (j) | |
| $C_4F_9$ | N,N-diméthyl dodécylamine | 89 | 19,0 (l) | |
| $C_6F_{13}$ | idem | 93 | 16,9 (j) | |
| $C_4F_9$ | N-allyl N-méthyl octylamine | 98 | 16,6 (k) | |
| $C_6F_{13}$ | idem | 78 | 15,1 (k) | |
| $C_4F_9$ | N-allyl N-méthyl nonylamine | 71 | 16,8 (k) | |
| $C_6F_{13}$ | idem | - | 16,1 (k) | |
| $C_4F_9$ | N-allyl N-méthyl décylamine | - | 17,4 (k) | |
| $C_6F_{13}$ | idem | - | 15,8 (k) | |
| $C_4F_9$ | N-allyl N-méthyl undécylamine | - | 18,5 (k) | |
| $C_6F_{13}$ | idem | 74 | 17,1 (k) | |
| $C_4F_9$ | N-allyl N-méthyl dodécylamine | 91 | 19,7 (k) | |
| $C_6F_{13}$ | idem | 84 | 18,0 (k) | |
| $C_4F_9$ | $C_4F_9C_2H_4SC_2H_4N(CH_3)_2$ | 92 | | |
| $C_6F_{13}$ | idem | 95 | | |
| $C_4F_9$ | $C_6F_{13}C_2H_4SC_2H_4N(CH_3)_2$ | 90 | | |
| $C_6F_{13}$ | idem | 91 | | |
| $C_6F_{13}$ | $C_8F_{17}C_2H_4SC_2H_4N(CH_3)_2$ | 90 | | |

TABLEAU B

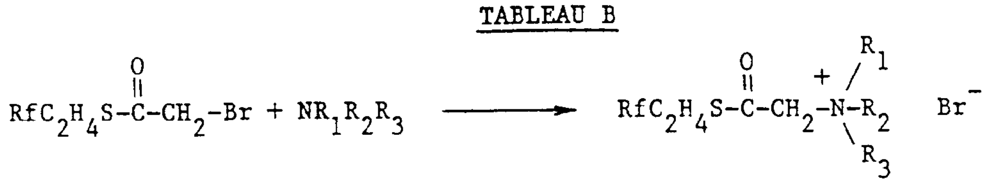

(I-b)

| Rf | Amine $NR_1R_2R_3$ | Rendement (%) | COMPOSE I-b γs | COMPOSE I-b γi |
|---|---|---|---|---|
| $C_4F_9$ | N,N-diméthyl heptylamine | 92 | 22,5 | 5,9 |
| $C_6F_{13}$ | idem | 91 | 17,2 | 3,2 |
| $C_8F_{17}$ | idem | 72 | 18,8 | 6,1 |
| $C_4F_9$ | N,N-diméthyl octylamine | 93 | 17,2 (k) | |
| $C_6F_{13}$ | idem | 95 | 16,5 (i) | |
| $C_8F_{17}$ | idem | 70 | 17,0 | |
| $C_4F_9$ | N,N-diméthyl nonylamine | 88 | 17,1 | 1,0 |
| $C_6F_{13}$ | idem | 91 | 16,8 | 3,0 |
| $C_8F_{17}$ | idem | 75 | 17,7 | |
| $C_4F_9$ | N,N-diméthyl décylamine | 93 | 18,7 | |
| $C_6F_{13}$ | idem | 89 | 18,9 | |
| $C_8F_{17}$ | idem | 78 | 15,8 (k) | |
| $C_4F_9$ | N,N-diméthyl dodécylamine | 91 | 18,7 | 0,7 |
| $C_6F_{13}$ | idem | 92 | 16,5 | 0,7 |
| $C_8F_{17}$ | idem | 72 | 16,0 (k) | |
| $C_4F_9$ | N-allyl N-méthyl octylamine | 70 | 17,6 (k) | |
| $C_6F_{13}$ | idem | 74 | 16,4 (k) | 0,8 |
| $C_8F_{17}$ | idem | 66 | 15,6 (k) | |

TABLEAU B (suite)

| Rf | Amine $NR_1R_2R_3$ | Rendement (%) | COMPOSE I-b γs | COMPOSE I-b γi |
|---|---|---|---|---|
| $C_4F_9$ | N-allyl N-méthyl nonylamine | 72 | 17,6 (k) | |
| $C_6F_{13}$ | idem | 73 | 16,1 (k) | |
| $C_8F_{17}$ | idem | 65 | 16,5 (k) | |
| $C_4F_9$ | N-allyl N-méthyl décylamine | 68 | 18,1 (k) | |
| $C_6F_{13}$ | idem | 72 | 16,0 (k) | |
| $C_4F_9$ | N-allyl N-méthyl undécylamine | 70 | 18,5 (k) | |
| $C_6F_{13}$ | idem | 71 | 16,3 (k) | |
| $C_4F_9$ | N-allyl N-méthyl dodécylamine | 67 | 19,1 (k) | |
| $C_6F_{13}$ | idem | 69 | 16,7 (k) | |
| $C_4F_9$ | $C_4F_9C_2H_4SC_2H_4N(CH_3)_2$ | 85 | | |
| $C_6F_{13}$ | idem | 81 | | |
| $C_4F_9$ | $C_6F_{13}C_2H_4SC_2H_4N(CH_3)_2$ | 77 | | |
| $C_6F_{13}$ | idem | 79 | 16,0 | 6,0 |
| $C_8F_{17}$ | idem | 80 | | |
| $C_4F_9$ | $C_8F_{17}C_2H_4SC_2H_4N(CH_3)_2$ | 65 | | |
| $C_6F_{13}$ | idem | 70 | | |
| $C_8F_{17}$ | idem | 70 | | |
| $C_4F_9$ | N-méthyl dioctylamine | 86 | | |
| $C_6F_{13}$ | idem | 85 | | |

TABLEAU B (suite)

| Rf | Amine $NR_1R_2R_3$ | COMPOSE I-b Rendement (%) | $\gamma s$ | $\gamma i$ |
|---|---|---|---|---|
| $C_4F_9$ | Méthacrylate de diméthyl-amino-2 éthyle | 88 | | |
| $C_6F_{13}$ | idem | 91 | | |
| $C_8F_{17}$ | idem | 65 | | |

## TABLEAU C

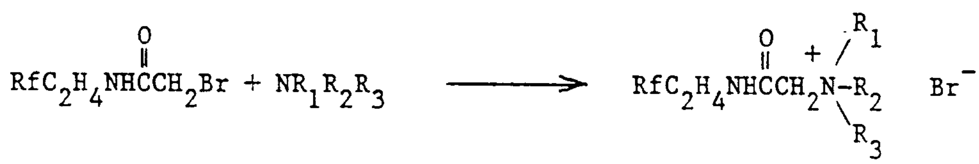

(I-c)

| Rf | Amine $NR_1R_2R_3$ | COMPOSE I-c Rendement (%) | $\gamma s$ | $\gamma i$ |
|---|---|---|---|---|
| $C_6F_{13}$ | N,N-diméthyl heptylamine | 88 | 15,4 | 1,5 |
| $C_4F_9$ | N,N-diméthyl octylamine | - | 17,6 | 0,4 |
| $C_6F_{13}$ | idem | 92 | 15,2 | 1,3 |
| $C_6F_{13}$ | N,N-diméthyl nonylamine | 82 | 15 | 1,2 |
| $C_4F_9$ | N,N-diméthyl décylamine | - | | |
| $C_6F_{13}$ | idem | 87 | 15,4 | 1,0 |
| $C_4F_9$ | N,N-diméthyl dodécylamine | - | | |
| $C_6F_{13}$ | idem | 86 | 16,8 | 0,4 |

Les N-allyl N-méthyl alkylamines de formule générale :

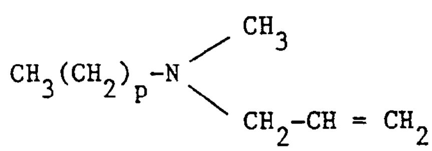

utilisés à l'exemple 5 ont été préparés en deux étapes, la première consistant à faire réagir l'allylamine $CH_2$ = $CH-CH_2-NH_2$ en excès sur le bromure d'alkyle correspondant $CH_3(CH_2)_p$-Br et la seconde à méthyler la N-allyl alkylamine ainsi obtenue $CH_3(CH_2)_p-NH-CH_2CH = CH_2$ au moyen d'un mélange formaldéhyde/acide formique. Le mode opératoire est le suivant:

a) Dans un réacteur muni d'un agitateur magnétique, d'un réfrigérant et d'une ampoule à brome et refroidi par un bain de glace, on place 0,3 mole d'allylamine, puis on ajoute lentement 0,1 mole de bromure d'alkyle et on chauffe ensuite à 50°C pendant 12 heures sous agitation. Après addition d'une solution aqueuse de soude saturée en chlorure de sodium, on extrait la phase organique à l'éther éthylique, puis on sèche la phase éthérée sur sulfate de sodium anhydre et évapore le solvant. Par distillation du résidu, on obtient la N-allyl alkylamine désirée et on récupère 0,2 mole d'allylamine qui peut être recyclée.

Le tableau D suivant donne le rendement et le point d'ébullition des différentes N-allyl alkylamines $CH_3(CH_2)_p-NH-CH_2CH=CH_2$ ainsi obtenues :

TABLEAU D

| p | 7 | 8 | 9 | 10 | 11 |
|---|---|---|---|---|---|
| Eb. sous 133 Pa | 61°C | 67°C | 75°C | 84°C | 101°C |
| Rdt. | 80 % | 75 % | 77 % | 73 % | 80 % |

b) Dans un réacteur muni d'un réfrigérant, d'une ampoule à brome et refroidi à 0°C, on introduit 0,01 mole de N-allyl alkylamine, puis on ajoute successivement et lentement 0,05 mole d'acide formique et 0,03 mole de formaldéhyde commercial en solution aqueuse à 35 %. On chauffe à 95-100°C pendant 12 heures, puis après retour à la température ambiante, on ajoute une solution aqueuse de soude saturée en chlorure de sodium. La phase organique est ensuite extraite à l'éther éthylique, puis séchée sur sulfate de sodium. Le solvant est évaporé et le résidu est distillé sous pression réduite pour recueillir la N-allyl N-méthyl alkylamine :

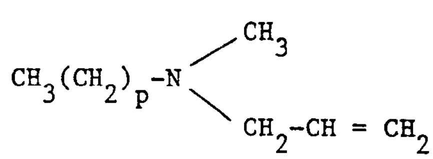

Le tableau suivant donne le rendement et le point d'ébullition des différentes N-allyl N-méthyl alkylamines ainsi obtenues :

TABLEAU E

| p | 7 | 8 | 9 | 10 | 11 |
|---|---|---|---|---|---|
| Eb. sous 133 pa | 56°C | 58°C | 72°C | 79°C | 99°C |
| Rdt. | 98 % | 99 % | 90 % | 95 % | 97 % |

EXEMPLE 6

On opère comme à l'exemple 5 (premier cas) en utilisant comme amine la N,N,N',N'-tétraméthyl éthylènediamine et comme bromure le bromoacétamide de perfluorohexyl-2 éthyle : $C_6F_{13}$-$C_2H_4$-NH-CO-$CH_2$-Br, avec un rapport molaire bromure/diamine égal à 2. Dans ces conditions on obtient avec un rendement de 91 % le sel double de formule :

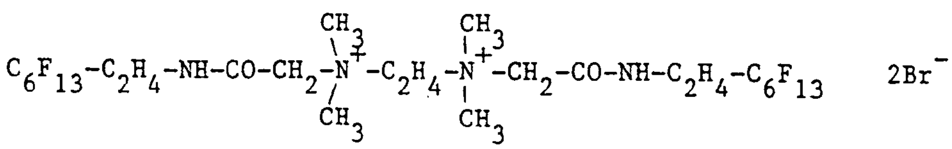

qui fond à 215°C. γs = 14,8 mN/m (sol. aq. 0,1 %).

En utilisant le bromoacétamide de perfluorobutyl-2 éthyle : $C_4F_9$-$C_2H_4$-NH-CO-$CH_2$-Br, on obtient le sel double suivant :

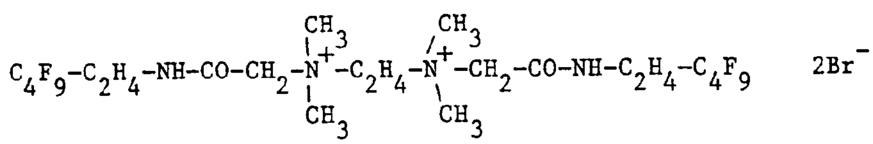

F = 210°C Rdt = 87 % γ.s = 20,1 mN/m (Sol. aq. 0,1 %).

## Revendications

### Revendications pour les etats contractants suivants : BE, CH, DE, FR, GB, IT, LI

1. Composés polyfluorés caractérisés en ce qu'ils répondent à la formule générale :

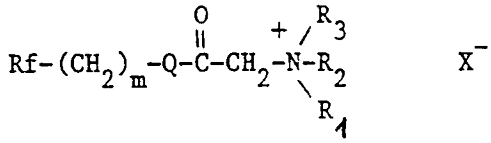

(I)

dans laquelle Rf représente un radical perfluoroalkyle, linéaire ou ramifié, contenant de 2 à 16 atomes de carbone, m est un nombre entier allant de 1 à 4, Q désigne un atome d'oxygène ou de soufre ou un groupe NH, $X^-$ représente un anion monovalent ou son équivalent, $R_1$ représente un radical alkyle contenant de 1 à 3 atomes de carbone, $R_2$ représente le radical allyle ou un radical alkyle, linéaire ou ramifié, contenant de 1 à 18 atomes de carbone, et $R_3$ représente un radical alkyle, linéaire ou ramifié, contenant de 7 à 18 atomes de carbone ou l'un des radicaux suivants :

$$-(CH_2)_n-S-(CH_2)_m-Rf' \qquad (II)$$

$$-(CH_2)_n\text{-O-CO-C(R)} = CH_2 \qquad \text{(III)}$$

```
      + /R1
  - Y-N---- CH2-CO-Q-(CH2)m-Rf     X-                    (IV)
      \
       R2
```

dans lesquels les symboles m, $R_1$, $R_2$, Q, Rf et $X^-$ sont tels que définis ci-dessus, n est un nombre entier allant de 2 à 4, R représente un atome d'hydrogène ou un radical méthyle, Y désigne un pont alkylène de 2 à 8 atomes de carbone éventuellement interrompu par un atome d'oxygène, et Rf' représente un radical perfluoroalkyle tel que Rf.

2. Composés polyfluorés selon la revendication 1, dans lesquels m est égal à 2.

3. Composés polyfluorés selon la revendication 1 ou 2, dans lesquels Rf et Rf' sont des radicaux perfluoroalkyle linéaires contenant 4, 6, 8 ou 10 atomes de carbone.

4. Procédé de préparation des composés polyfluorés selon l'une des revendications 1 à 3, caractérisé en ce que l'on fait réagir un halogéno-acétate, -thioacétate ou -acétamide de formule :

```
                  O
                  ||
Rf-(CH2)m-Q-C-CH2-Cl (ou Br)                    (V)
```

avec l'amine tertiaire correspondante et, éventuellement, on échange l'anion chlorure ou bromure du sel obtenu contre un autre anion.

5. Utilisation des composés polyfluorés selon l'une des revendications 1 à 3 comme agents tensio-actifs ou comme monomères pour la fabrication de vésicules artificielles.

**Revendications pour l'Etat contractant suivant : ES**

1. Procédé de préparation des composés polyfluorés répondant à la formule générale :

```
               O         R3
               ||      + /
Rf-(CH2)m-Q-C-CH2-N-R2            X-              (I)
                         \
                          R1
```

dans laquelle Rf représente un radical perfluoroallyle, linéaire ou ramifié, contenant de 2 à 16 atomes de carbone, m est un nombre entier allant de 1 à 4, Q désigne un atome d'oxygène ou de soufre ou un groupe NH, $X^-$ représente un anion monovalent ou son équivalent, $R_1$ représente un radical allyle contenant de 1 à 3 atomes de carbone, $R_2$ représente le radical allyle ou un radical allyle, linéaire ou ramifié, contenant de 1 à 18 atomes de carbone, et $R_3$ représente un radical allyle, linéaire ou ramifié, contenant de 7 à 18 atomes de carbone ou l'un des radicaux suivants :

$$-(CH_2)_n\text{-S-}(CH_2)_m\text{-Rf'} \qquad \text{(II)}$$
$$-(CH_2)_n\text{-O-CO-C(R)} = CH_2 \qquad \text{(III)}$$

```
       R1
     + /
 -Y-N---CH2-CO-Q-(CH2)m-Rf       X-              (IV)
     \
      R2
```

dans lesquels les symboles m, $R_1$, $R_2$, Q, Rf et $X^-$ sont tels que définis ci-dessus, n est un nombre entier allant de 2 à 4, R représente un atomes d'hydrogène ou un radical méthyle, Y désigne un pont allylène de 2 à 8 atomes de carbone éventuellement interrompu par un atome d'oxygène, et Rf' représente un radical perfluoroallyle tel que Rf, caractérisé en ce que l'on fait réagir un halogéno-acétate, -thioacétate ou -acétamide de formule :

$$Rf-(CH_2)_m-Q-\overset{\overset{O}{\|}}{C}-CH_2-Cl \text{ (ou Br)} \qquad (V)$$

avec l'amine tertiaire correspondante et, éventuellement, on échange l'anion chlorure ou bromure du sel obtenu contre un autre anion.

2. Procédé selon la revendication 1, dans lequel m est égal à 2.

3. Procédé selon la revendication 1 ou 2, dans lequel Rf et Rf′ sont des radicaux perfluoroallyle linéaires contenant 4, 6, 8 ou 10 atomes de carbone.

4. Utilisation des composés polyfluorés (I) tels que définis dans la revendication 1 comme agents tensioactifs ou comme monomères pour la fabrication de vésicules artificielles.

## Patentansprüche

### Patentansprüche für folgende Verstragsstaaten : BE, CH, DE, FR, GB, IT, LI

1. Polyfluorierte Verbindungen, dadurch gekennzeichnet, daß sie der allgemeinen Formel

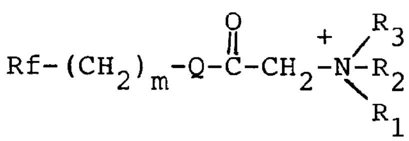

$X^-$ (I)

entsprechen, in der Rf einen geradkettigen oder verzweigten Perfluoralkylrest mit 2 bis 16 Kohlenstoffatomen bedeutet, m eine ganze Zahl von 1 bis 4 ist, Q ein Sauerstoff- oder ein Schwefelatom oder eine NH Gruppe bezeichnet, $X^-$ ein einwertiges Anion oder sein Äquivalent bedeutet, $R_1$ einen Alkylrest mit 1 bis 3 Kohlenstoffatomen bedeutet, $R_2$ einen Allylrest oder einen geradkettigen oder verzweigten Alkylrest mit 1 bis 18 Kohlenstoffatomen und $R_3$ einen geradkettigen oder verzweigten Alkylrest mit 7 bis 18 Kohlenstoffatomen bedeutet oder einer der folgenden Reste ist:

$$-(CH_2)_n-S-(CH_2)_m-Rf' \qquad (II)$$

$$-(CH_2)_n-O-CO-C(R)=CH_2 \qquad (III)$$

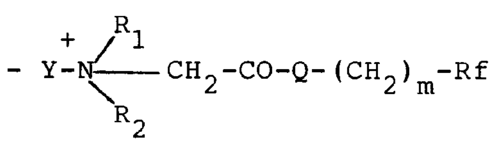

$X^-$ (IV)

in denen die Symbole m, $R_1$, $R_2$, Q, Rf und $X^-$ wie oben definiert sind, n eine ganze Zahl von 2 bis 4 ist, R ein Wasserstoffatom oder einen Methylrest bedeutet, Y eine Alkylenbrücke mit 2 bis 8 Kohlenstoffatomen bedeutet, die gegebenenfalls durch ein Sauerstoffatom unterbrochen ist, und Rf′ einen Perfluoralkylrest wie Rf bedeutet.

2. Polyfluorierte Verbindungen nach Anspruch 1, in denen m gleich 2 ist.

3. Polyfluorierte Verbindungen nach Anspruch 1 oder 2, in denen Rf und Rf′ geradkettige Perfluoralkylreste mit 4, 6, 8 oder 10 Kohlenstoffatomen sind.

4. Verfahren zur Herstellung von polyfluorierten Verbindungen nach einem der Ansprüche 1 bis 3, dadurch gekennzeichnet, daß man ein Halogenacetat, -thioacetat oder -acetamid der Formel:

$$Rf-(CH_2)_m-Q-\overset{\overset{O}{\|}}{C}-CH_2-Cl \text{ (oder Br)} \qquad (V)$$

mit einem entsprechenden tertiären Amin umsetzt und gegebenenfalls das Chlorid- oder das Bromidanion des erhaltenen Salzes gegen ein anderes Anion austauscht.

5. Verwendung der polyfluorierten Verbindungen nach einem der Ansprüche 1 bis 3 als oberflächenaktive Mittel oder als Monomere zur Herstellung von künstlichen Bläschen.

**Patentansprüche fur folgenden Vertragsstaat : ES**

1. Verfahren zur Herstellung von polyfluorierten Verbindungen, die der allgemeinen Formel

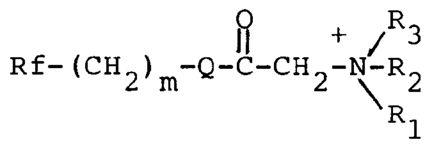 $X^-$ (I)

entsprechen, in der Rf einen geradkettigen oder verzweigten Perfluoralkylrest mit 2 bis 16 Kohlenstoffatomen bedeutet, m eine ganze Zahl von 1 bis 4 ist, Q ein Sauerstoff- oder Schwefelatom oder eine NH Gruppe bezeichnet, $X^-$ ein einwertiges Anion oder sein Äquivalent bedeutet, $R_1$ einen Alkylrest mit 1 bis 3 Kohlenstoffatomen bedeutet, $R_2$ einen Allylrest oder einen geradkettigen oder verzweigten Alkylrest mit 1 bis 18 Kohlenstoffatomen bedeutet und $R_3$ einen geradkettigen oder verzweigten Alkylrest mit 7 bis 18 Kohlenstoffatomen bedeutet oder einer der folgenden Reste ist:

$$-(CH_2)_n-S-(CH_2)_m-Rf' \quad (II)$$

$$-(CH_2)_n-O-CO-C(R)=CH_2 \quad (III)$$

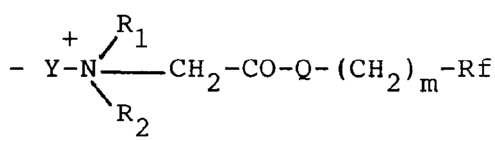 $X^-$ (IV)

in denen die Symbole m, $R_1$, $R_2$, Q, Rf und $X^-$ wie oben definiert sind, n eine ganze Zahl von 2 bis 4 ist, R ein Wasserstoffatom oder einen Methylrest bedeutet, Y eine Alkylenbrücke mit 2 bis 8 Kohlenstoffatomen bedeutet, die gegebenenfalls durch ein Sauerstoffatom unterbrochen ist, und Rf' einen Perfluoralkylrest wie Rf bedeutet, dadurch gekennzeichnet, daß man ein Halogenacetat, -thioacetat oder -acetamid der Formel:

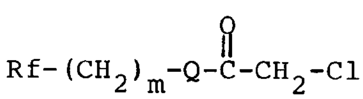 (oder Br) (V)

mit einem entsprechenden tertiären Amin umsetzt und gegebenenfalls das Chlorid- oder das Bromidanion des erhaltenen Salzes gegen ein anderes Anion austauscht.

2. Verfahren nach Anspruch 1, in dem m gleich 2 ist.

3. Verfahren nach Anspruch 1 oder 2, in dem Rf und Rf' geradkettige Perfluoralkylreste mit 4, 6, 8 oder 10 Kohlenstoffatomen sind.

4. Verwendung der polyfluorierten Verbindungen (I) nach Anspruch 1 als oberflächenaktive Mittel oder als Monomere zur Herstellung von künstlichen Bläschen.

## Claims

**Claims for the following Contracting States : BE, CH, DE, FR, GB, IT, LI**

1. Polyfluorinated compounds characterised in that they correspond to the general formula:

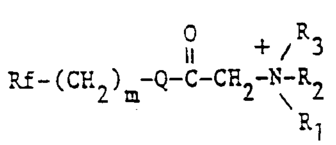 $X^-$ (I)

in which Rf denotes a linear or branched perfluoroalkyl radical containing from 2 to 16 carbon atoms, m is an

integer ranging from 1 to 4, Q denotes an oxygen or sulphur atom or an NH group, $X^-$ denotes a monovalent anion or its equivalent, $R_1$ denotes an alkyl radical containing from 1 to 3 carbon atoms, $R_2$ denotes the allyl radical or a linear or branched alkyl radical containing from 1 to 18 carbon atoms, and $R_3$ denotes a linear or branched alkyl radical containing from 7 to 18 carbon atoms or one of the following radicals:

$$-(CH_2)_n-S-(CH_2)_m-Rf' \qquad (II)$$

$$-(CH_2)_n-O-CO-C(R)=CH_2 \qquad (III)$$

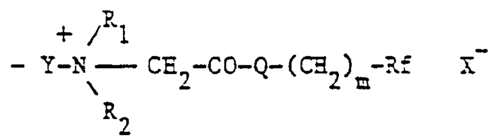

(IV)

in which the symbols m, $R_1$, $R_2$, Q, Rf and $X^-$ are as defined above, n is an integer ranging from 2 to 4, R denotes a hydrogen atom or a methyl radical, Y denotes an alkylene bridge of 2 to 8 carbon atoms optionally interrupted by an oxygen atom, and Rf' denotes a perfluoroalkyl radical such as Rf.

2. Polyfluorinated compounds according to Claim 1, in which m is equal to 2.

3. Polyfluorinated compounds according to Claim 1 or 2, in which Rf and Rf' are linear perfluoroalkyl radicals containing 4, 6, 8 or 10 carbon atoms.

4. Process for the preparation of the polyfluorinated compounds according to one of Claims 1 to 3, characterised in that a haloacetate, -thioacetate or -acetamide of formula:

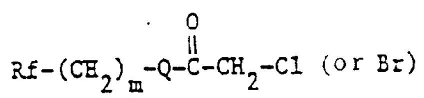

(V)

is reacted with the corresponding tertiary amine and, optionally, the chloride or bromide anion of the salt obtained is exchanged for another anion.

5. Use of the polyfluorinated compounds according to one of Claims 1 to 3 as surface-active agents or as monomers for the manufacture of artificial vesicles.

**Claims for the following Contracting State : ES**

1. Process for the preparation of the polyfluorinated compounds corresponding to the general formula:

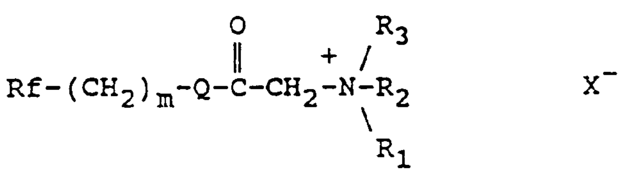

(I)

in which Rf denotes a linear or branched perfluoroalkyl radical containing from 2 to 16 carbon atoms, m is an integer ranging from 1 to 4, Q denotes an oxygen or sulphur atom or an NH group, $X^-$ denotes a monovalent anion or its equivalent, $R_1$ denotes an alkyl radical containing from 1 to 3 carbon atoms, $R_2$ denotes the allyl radical or a linear or branched alkyl radical containing from 1 to 18 carbon atoms, and $R_3$ denotes a linear or branched alkyl radical containing from 7 to 18 carbon atoms or one of the following radicals:

$$-(CH_2)_n-S-(CH_2)_m-Rf' \qquad (II)$$

$$-(CH_2)_n-O-CO-C(R)=CH_2 \qquad (III)$$

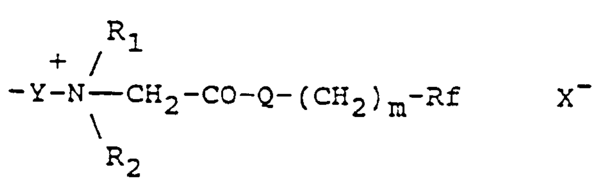

(III)

(IV)

in which the symbols m, $R_1$, $R_2$, Q, Rf and $X^-$ are as defined above, n is an integer ranging from 2 to 4, R denotes a hydrogen atom or a methyl radical, Y denotes an alkylene bridge of 2 to 8 carbon atoms optionally interrupted by an oxygen atom, and Rf′ denotes a perfluoroalkyl radical such as Rf, characterised in that a haloacetate, -thioacetate or -acetamide of formula:

$$Rf-(CH_2)_m-Q-\overset{O}{\overset{\|}{C}}-CH_2-Cl \text{ (or Br)} \qquad (V)$$

is reacted with the corresponding tertiary amine and, optionally, the chloride or bromide anion of the salt obtained is exchanged for another anion.

2. Process according to Claim 1, in which m is equal to 2.

3. Process according to Claim 1 or 2, in which Rf and Rf′ are linear perfluoroalkyl radicals containing 4, 6, 8 or 10 carbon atoms.

4. Use of the polyfluorinated compounds (I) as defined in Claim 1 as surface-active agents or as monomers for the manufacture of artificial vesicles.